# EUROPEAN PATENT APPLICATION

(11) **EP 4 001 399 A1**
(43) Date of publication of application: **25.05.2022**
(21) Application number: 20020530.0
(22) Date of filing: 13.11.2020
(51) Int. Cl.: C12N 1/20, A23L 33/135, A61K 35/745, C12R 1/01

(54) **STRAIN BIFIDOBACTERIUM ANIMALIS SSP. LACTIS DSM 33647 FOR MODULATING METABOLISM INDICATORS**

(71) Applicant: "NEOPHARM BULGARIA" Ltd., 1408 Sofia (BG)
(72) Inventor: Drentchev, Petko, 1000 Sofia (BG); Stoyanov, Stanislav, 4000 Plovdiv (BG); Pavlov, Atanas, 4000 Plovdiv (BG); Tchorbanov, Andrei, 1000 Sofia (BG)
(74) Representative: Stefanova, Stanislava Hristova

(57) **Abstract**

The invention relates to a strain *Bifidobacterium animalis ssp. lactis* DSM 33647, applicable for modulation of key metabolic parameters, necessary for preventing the development of metabolic syndrome, in particular body mass index, lipid metabolism and sugar metabolism.

## Description

### Field of the invention

The invention relates to a strain *Bifidobacterium animalis ssp. lactis* DSM 33647, applicable for modulation of key metabolic parameters, necessary for preventing the development of metabolic syndrome, in particular body mass index, lipid metabolism and sugar metabolism.

### Background of the Invention

It is known that many strains of *Bifidobacterium animalis* ssp. *lactis* are considered probiotic microorganisms and are widely used in various fermented or unfermented foods. Characteristics that make strains of this subspecies desirable for use as probiotics include the understanding of the health benefits and technological advantages over organisms of the same genus as modulating the immune system (1) and increased digestive comfort as well as oxygen tolerance, acid resistance and bile, as well as viability during prolonged storage in refrigerated conditions.

For example, *B. animalis ssp. lactis* DSM 10140, first described in 1997 as a unique strain of *Bifidobacterium,* has been identified as an oxygen tolerant isolate from a sample of fermented milk (2), while studies of a probiotic strain of *B. animalis ssp. Lactis* LKM512 have shown that it reduces the risk of developing atherosclerosis in healthy people by reducing trimethylamine (TMA) produced by the intestinal microbiota (3).

In addition, according to patent US2009170185, a combination of strains of *B. animalis ssp. animalis* YIT10394, *B. animalis ssp. Lactis* JCM 1253 and *B. animalis ssp. lactis* JCM 7117 shows an inhibitory effect on cholesterol absorption in the intestinal tract.

The known solutions, however, focus mainly on the activity of the strains belonging to the species of *Bifidobacterium animalis* ssp. *lactis* in terms of cholesterol levels and the risk of developing atherosclerosis.

### Summary of the Invention

The invention relates to a strain *Bifidobacterium animalis ssp. lactis,* registered with the German Cell Culture Bank (DSMZ) under number DSM33647, applicable for modulation of key metabolic parameters, necessary for preventing the development of metabolic syndrome, in particular body mass index, lipid metabolism and sugar metabolism.

The strain is isolated from breast milk in a selective culture medium BSM (Bifidobacterium Selective Medium). The source from which the strain is isolated is based on the knowledge that the microorganisms obtained through breast milk are carriers of beneficial properties that are passed down through generations from healthy mothers to their children.

Morphologically, the strain *B. animalis ssp. lactis* DSM33647 is characterized by elongated cells with rounded ends, growing singly, in pairs and in short chains. Its colonies are 1.0-1.5 mm in size, convex, beige, glossy, with a regular end. It is a Gram-positive, catalase-negative strain fermenting glucose, lactose, sucrose, maltose, salicin, xylose, arabinose, mannose and raffinose, and hydrolyzing gelatin and aesculin. The strain exhibits esterase, esterase-lipase, leucine arylamidase, phosphatase, phosphohydrolase, α- and β- galactosidase as well as α- 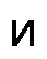 β- glucosidase activity detected by API 20A and API ZYM assay.

The main probiotic properties of the strain, according to the invention, were demonstrated under conditions of a simulated gastrointestinal tract in terms of acid tolerance, resistance to gastric and pancreatic juices and resistance to bile salts, shown in Table 1 below. Especially indicative of survival of the strain of *B. animalis ssp. lactis* DSM33647 in an acidic gastric environment is its survival at pH 3 - it is greater than 90%. Experiments with different concentrations of bile acid also show a high survival of the strain - over 90% and when treated with bile salts at a concentration of 2.0%.

A study performed on a mouse model of oral feeding of animals with the strain of *B*. *animalis ssp. lactis* DSM33647 proves beyond doubt that the strain has a strong adhesion ability, as well as the ability to colonize the intestine, which allows it to remain on the lining of the colon even 3 days after the end of feeding. This once again supports the conclusion of high survival of the strain according to the invention in the conditions of the gastrointestinal tract.

**TABLE 1**

| | Survival of the strain DSM33647 |
|---|---|
| Acid tolerance | High (> 90%) survival at pH 3.0 for 3h at 37°C; > 50% survival at pH 1.5 for 3 h at 37°C |
| Resistance to bile salts | High (> 90%) survival when treated with 2.0% bile salts for 2h at 37°C |
| Resistance to gastric juices | High (> 80%) survival in simulated gastric juices for 3 hours at 37°C |
| Resistance to pancreatic juices | The strain retains viability in a medium of simulated pancreatic juices for 4 h at 37°C |

The antibiotic susceptibility of the strain B. animalis ssp. lactis DSM33647 has been investigated. The minimum inhibitory concentration (MIC) in µg/ml of 14 antibiotics is established: ampicillin, vancomycin, gentamicin, streptomycin, erythromycin, clindamycin, tetracycline, chloramphenicol, kanamycin, ciprofloxacin, linezolid, rifampicin, trimethoprim, penicillin. MIC data are summarized in Table 2 below. In addition, the strain of *B. animalis ssp. lactis* DSM33647 is resistant to streptomycin and sensitive to ampicillin, vancomycin, tetracycline, erythromycin, clindamycin, chloramphenicol and gentamicin as determined by EFSA Scientific Opinion (4).

**TABLE 2**

| | Antibiotic | MIC for the strain of DSM33647, µg/ml | EFSA limit values for *Bifidobacterium* (µg/ml) | Sensitive (S)/ Resistant (R) |
|---|---|---|---|---|
| 1 | ampicillin | 0.75 | 2 | S |
| 2 | vancomycin | 2.0 | 2 | S |
| 3 | tetracycline | 0.38 | 8 | S |
| 4 | erythromycin | 0.75 | 1 | S |
| 5 | clindamycin | 0.75 | 1 | S |
| 6 | streptomycin | >256 | 128 | R |
| 7 | chloramphenicol | 3.0 | 4 | S |
| 8 | kanamycin | >256 | - | - |
| 9 | gentamicin | 32.0 | 64 | S |
| 10 | ciprofloxacin | 0.75 | / | / |
| 11 | linezolid | 1.5 | / | / |
| 12 | rifampicin | 0.5 | / | / |
| 13 | trimethoprim | >32 | / | / |
| 14 | penicillin | 2.0 | / | / |

Quantitative studies on the inhibitory effect of the strain *Bifidobacterium animalis ssp. Lactis* DSM33647 on pathogenic microorganisms of the species *Firmicutes* (*Enterococcus faecium*) and *Bacteroidetes* (*Bacteroides fragilis)* show a significant decrease in their number in experiments *in vitro.* This proves the quality of the strain of *Bifidobacterium animalis ssp. lactis* DSM33647 to produce bacteriocins that attack the pathogenic microflora in the body in various inflammatory processes. Statistical processing of the results shows values of the average statistical deviation *p<0.01.*

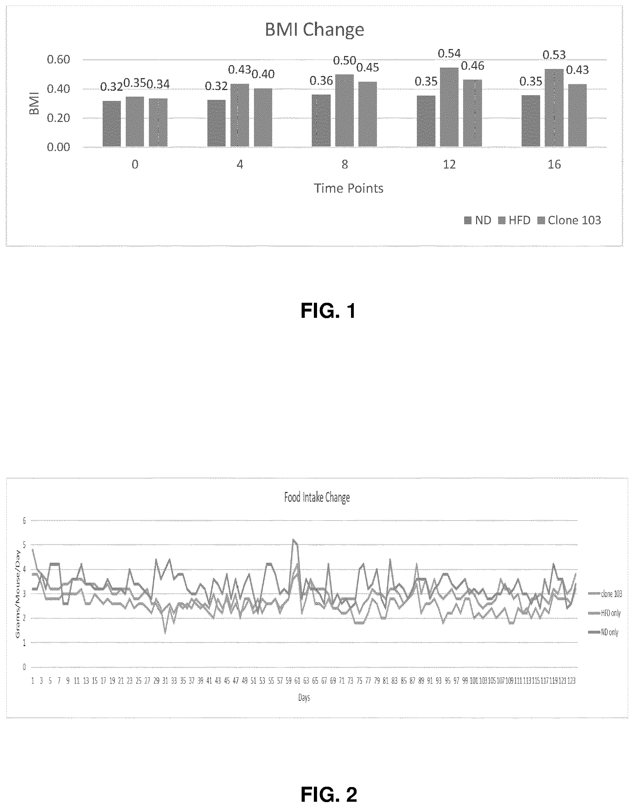

The strain, according to the invention, is subjected to studies aimed at determining its influence on certain metabolic parameters, which are key parameters to preventing the development of metabolic syndrome. The studies have been performed on an animal model for the development of metabolic syndrome in the mouse line C57BL/6J. Growth, weight, body mass index (BMI), blood sugar, plasma insulin, insulin resistance, triglycerides, total cholesterol, high-density lipoprotein (HDL), low-density lipoprotein (LDL) and liposaccharides (LPS) were observed and beta-cell function. The results obtained, described in detail in Examples 2 to 5 and illustrated by Figures 1 to 9, unequivocally show that the strain *Bifidobacterium animalis ssp. lactis* DSM33647 positively affects the observed indicators. Of particular note are the results regarding the values of lipopolysaccharides (LPS) in the blood plasma of the animals treated with the strain according to the invention, compared with the control group subjected to a high-fat diet (HFD). This indicator of metabolism reflects the permeability of the intestinal wall to large molecules and toxins. Decreases in permeability and LPS levels indicate the protective effect of the strain of DSM33647 against Leaky Gut Syndrome (LGS).

In addition, optimized conditions have been developed for cell proliferation of the strain according to the invention and their maximum survival both in the native state and in lyophilized, allowing the achievement of live cell content from the strain of *Bifidobacterium animalis ssp. lactis* DSM33647 between 3.1 x 10¹¹ and 3.4 x 10¹¹ CFU/ml.

The property of the strain *B. animalis ssp. lactis* DSM33647 to produce bacteriocins allows its use in the form of pharmaceutical or food products as a means against pathogenic microorganisms in the body in various inflammatory processes on the one hand, and its resistance to antibiotics allows its use in parallel with them or as a finishing healing process factor.

The properties of the strain *B. animalis ssp. lactis* DSM33647 to affect key metabolic parameters necessary for preventing the development of metabolic syndrome, on the other hand, allows its use as a probiotic product for oral administration in the form of tablets, capsules, dry oral supplements and others as a prophylactic, therapeutic and curative agent with a beneficial effect on metabolism in the human body.

### Description of the attached Figures:

Fig. 1 Shows Body Mass Index (BMI) values in treated with strains of DSM33647 and untreated animals
Fig. 2 Represents an analysis of food intake
Fig. 3 Represents the values from the measurement of triglycerides
Fig. 4 Represents HDL values in treated with the strain of DSM33647 and untreated animals
Fig. 5 Represents lipopolysaccharide levels in treated with the strain of DSM33647 and untreated animals
Fig. 6 Represents blood sugar levels in treated with the strain of DSM33647 and untreated animals
Fig. 7 Represents serum insulin levels in treated with the strain of DSM33647 versus untreated animals
Fig. 8 Represents the values of insulin resistance
Fig. 9 Represents beta-cell function results in treated with the strain of DSM33647 versus untreated animals

The invention is illustrated by the following Examples, without limiting the scope of its application:

### Example 1: Obtaining a biologically pure culture from a strain Bifidobacterium animalis ssp. lactis DSM33647

The strain has been isolated from breast milk in selective culture medium BSM (Bifidobacterium Selective Medium) and stored at -80°C in cryoprotective tubes, in media with 40% glycerol. Prior to the experiments, the isolate has been activated by repeated culture of solid and liquid MRS medium at 37°C under anaerobic conditions for 48 hours. The culture medium in which the largest number of cells accumulates in one cubic centimeter of culture fluid, the composition of which is as follows, is selected (g/L):
Glucose - 17.5; yeast extract - 15.0; casein- 15.0; peptone- 1.0; KH2PO4- 1.0; K2HPO4-1.0; MgSO4 . 7H2O- 0.2; MnSO4- 0.007; FeSO4- 0.01; Tween 80- 1.0; NaCI-0.01 and cysteine - 0.5.

The experiments have been conducted in cultivation banks with a total volume of 2 liters and a working volume of 1.6 liters, under static conditions. The anaerobic conditions of the experiment are provided by purging the culture medium with sterile nitrogen immediately before inoculation of the system, and during cultivation the cultivation banks are hermetically sealed. The titer of the cells obtained is 4.3 × 107 CFU/ml. From the primary culture thus obtained, subcultures have been prepared in order to increase the number of cells from the pure strain to obtain mother cultures, which could then be used as an inoculant in the industrial production of a bacterial culture from the strain *B*. *animalis ssp. lactis* DSM33647.

Subcultures have been prepared by periodic cultivation in a New Brunswick BioFlo 110 bioreactor at an active acidity of 5.5 and a cultivation temperature of 37°C. Under these cultivation conditions, about 10 times more cells accumulate - up to about 4.4x1011 CFU/ml. Subsequent lyophilization with the use of sorbitol as a cryoprotectant allows to achieve the content of living cells of the strain *B. animalis ssp.lactis* DSM33647 in an amount between 3.1 x 1011 and 3.4 x 1011 CFU/ml and survival 71%.

### Example 2: Tracking the effect of treatment with strain Bifidobacterium animalis ssp. lactis DSM33647 on body mass index values (BMI)

10-week-old non-sterile mice of both sexes, line C57BL/6J, kept in an isolated environment with free access to food and water in a 12-hour day/night cycle, have been divided into groups as follows:
- Negative control that eats normal food (ND) - food type Research Diets D12450H, for 16 weeks;
- Positive control on a high fat diet (HFD) - food type Research Diet D12451, for 16 weeks;
- Active group undergoing HFD and subsequent probiotic treatment with the strain of DSM33647 at a dose of 109 CFU/g, namely: 8 weeks HFD until induction of metabolic syndrome (25% weight gain corresponding to obesity Class II-BMI 30-35, moderate obesity) and 8 weeks HFD + Probiotic (ie after induction of metabolic syndrome).

The analysis of the results is performed during and at the end of the experiment in terms of weight and body mass index (BMI) of the animals, as well as in terms of the amount of food consumed.

The results show that all mice treated with the strain *B. animalis ssp.lactis* DSM33647 showed retention in the increase in BMI shown in Figure 1, where, as elsewhere in the attached figures, the strain *B. animalis ssp.lactis* DSM33647 is designated as branch 103. The difference with the HFD control group is quite clear, but the values never reach the levels of the group on a normal diet.

Weight gain results showed that the strain *B. animalis ssp.lactis* DSM33647 reduces the weight of mice with advanced metabolic syndrome, manifested by a statistically significant difference in weight and BMI with positive control of HFD.

Impressively, mice on a normal diet (ND) ate the most food, and the group treated with the strain of DSM33647 ate less food than HFD control animals without treatment (Figure 2).

### Example 3: Monitoring of lipid metabolism in the treatment of animals with the strain B. animalis ssp.lactis DSM33647

The indicators of lipid metabolism - cholesterol, triglycerides, LDL and HDL have been monitored in the groups of animals as in Example 2.

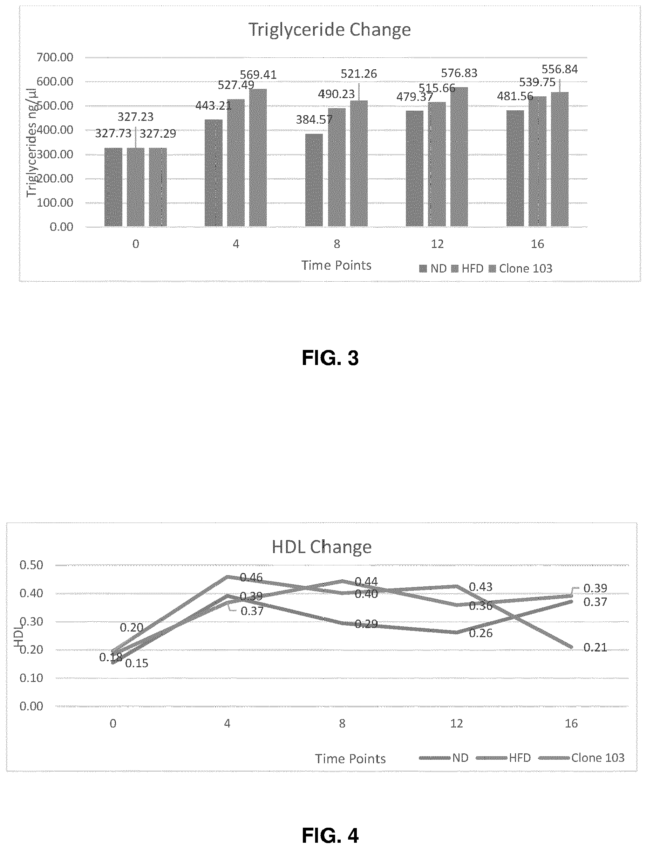

The results of the measurement of triglycerides do not show statistically significant differences from the treated and untreated with the strain, according to the invention, groups of animals subjected to a high-fat diet (Fig. 3). As the main source of triglycerides is food and the groups treated with the strain *B. animalis ssp.lactis* DSM33647 are HFD fed, the effect is expected and no drastic change in triglyceride levels is observed.

In contrast, the analysis of High Density Lipoprotein (HDL) showed an increase in values in animals treated with the strain *B. animalis ssp.lactis* DSM33647, as well as a tendency to maintain high plasma HDL values compared to the control group of animals subjected to high fat diet (HFD). The indicators are defined with Cholesterol Assay Kit (ab65390) Abcam, GB.

The results are illustrated in Figure 4, where the values for the strain of DSM33647 are designated as branch 103.

### Example 4: Monitoring of lipopolysaccharide levels in the treatment of animals with the strain of B. animalis ssp. lactis DSM33647

Lipopolysaccharide levels have been monitored in the animal groups as in Example 2, as determined by the Kinetic-QCL 192 Test Kit, Lonza lipopolysaccharide test.

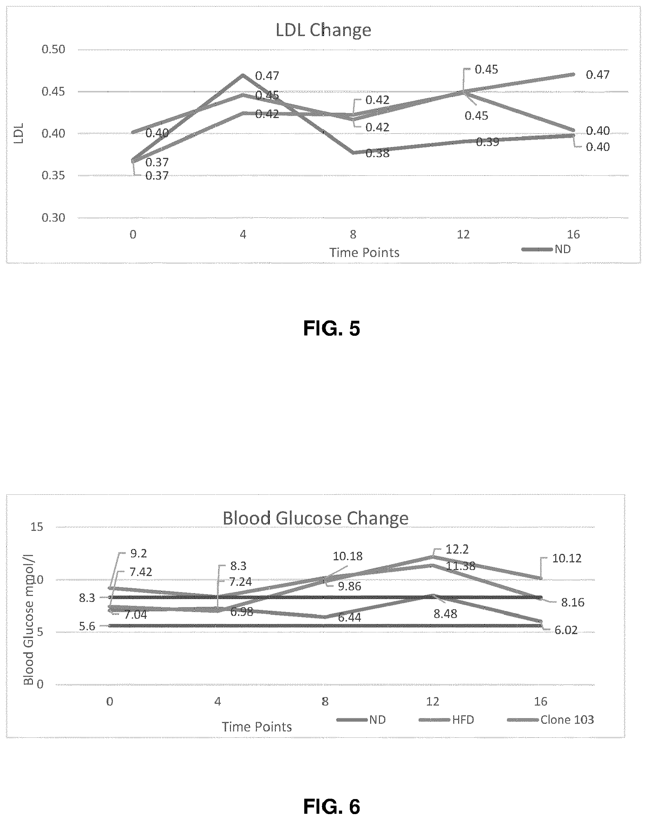

The test results illustrated in Figure 5 show that in the groups treated with *B. animalis ssp. Lactis* DSM33647, there is a decrease in serum lipopolysaccharide levels. Interestingly, lipopolysaccharide levels in the normal diet group (ND) eventually reached those fed with HFD.

In addition, monitoring of lipopolysaccharide levels reflects the permeability of the intestinal wall to large molecules and toxins. Decreases in permeability and lipopolysaccharide levels indicate the protective effect of the strain of DSM33647 against Leaky Gut Syndrome (LGS).

### Example 5: Tracking the effect of impact of the strain B. animalis ssp. lactisDSM33647 on sugar metabolism

To track the effect of impact of strain *B. animalis ssp. lactis* DSM33647 on sugar metabolism, indicators of blood glucose, insulin levels and insulin resistance and beta-cell function have been determined in animals treated with the strain of *B. animalis ssp. lactis* DSM33647.

As blood sugar levels are of particular importance for determining a possible pre-diabetic condition, plasma blood glucose levels have been monitored as in Example 2. The experiment is performed with standard automated tests. The results illustrated in Figure 6 show a statistically significant reduction in blood sugar levels in animals treated with strain *B. animalis ssp. lactis* DSM33647, according to the invention, by the 16th week of the experiment, compared to the control group subjected to a high-fat diet (HFD).

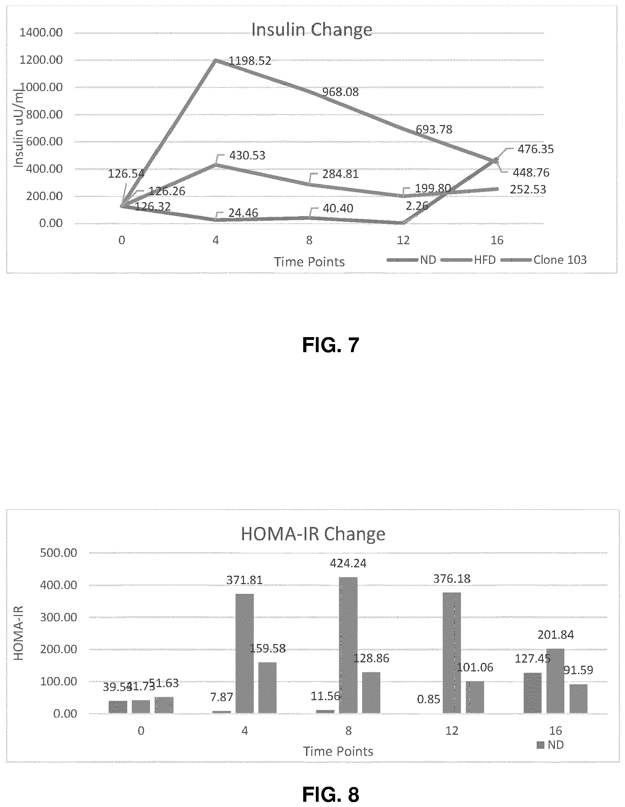

Serum insulin levels have been monitored in the same group of animals as in Example 2. Insulin levels in the sera of mice treated with the strain *B. animalis ssp. lactis* DSM33647 have been found to be significantly lower than those of the untreated control. This shows unequivocally that sugar metabolism is much better protected in the treatment with the bacteria, according to the invention. The results are illustrated in Figure 7.

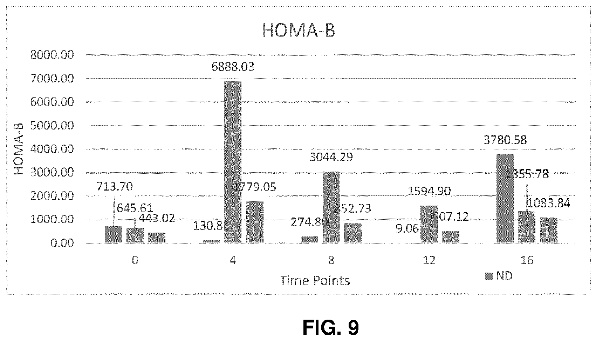

The insulin resistance and beta-cell function test in the experimental animals, as in Example 2, showed that the values decreased statistically significantly in the laboratory animals treated with the strain *B. animalis ssp. lactis* DSM33647 at week 16^{th}, compared to the HFD control (Figure 8). The results in terms of beta-cell function are particularly indicative - extremely intensive work is found in the untreated group, showing damage and the need for increased amounts of insulin, while the glands in the groups treated with the strain *B. animalis ssp. lactis* DSM33647 work much closer to the norm in animals, fed a normal diet (ND) (Figure 9).

### REFERENCES

1. Rizzardini G, Eskesen D, Calder PC, Capetti A, Jespersen L, Clerici M. 2012. Evaluation of the immune benefits of two probiotic strains Bifidobacterium animalis ssp. lactis, BB-12(R) and Lactobacillus paracasei ssp. paracasei, L. casei 431 (R) in an influenza vaccination model: a randomised, double-blind, placebo- controlled study. Br. J. Nutr. 107:876-884.
2. Meile L, Ludwig W, Reuger U, Gut C, Kaufmann P, Dasen G, WengerS, Teuber M. 1997. Bifidobacterium lactis sp. nov., a moderately oxygen tolerant species isolated from fermented milk. Syst. Appl. Microbiol. 20:57-64.
3. Mitsuharu M., at al., Bifidobacterium animalis ssp. lactis LKM512, Journal of Functional Foods, Volume 36, September 2017, Pages 94-101
4. Guidance on the assessment of bacterial susceptibility to antimicrobials of human and veterinary importance, EFSA Journal 2012;10(6):2740.

## Claims

1. Strain *Bifidobacterium animalis ssp. lactis,* registered in DSMZ under number DSM 33647.

2. Use of strain *Bifidobacterium animalis ssp. lactis* DSM 33647, according to Claim 1 for modulation of the metabolic parameters, necessary for preventing the development of metabolic syndrome.

3. Pharmaceutical and food type products according to Claims 1 and 2, **characterized in that** they comprise from 3.1 x 10¹¹ to 3.4 x 10¹¹ CFU/ml live cells of a biologically pure culture of the strain *Bifidobacterium animalis ssp. lactis* DSM 33647.
